# EUROPEAN PATENT APPLICATION

(11) **EP 3 207 939 A2**
(43) Date of publication of application: **23.08.2017**
(21) Application number: 15850491.0
(22) Date of filing: 15.09.2015
(51) Int. Cl.: A61K 38/17, A61K 35/30, A61K 35/60, A61P 9/10

(54) **USE OF A PROTEIN/PEPTIDE COMPLEX FOR TREATING AND PREVENTING ATHEROSCLEROSIS, METHOD FOR TREATING AND PREVENTING ATHEROSCLEROSIS (VARIANTS)**

(30) Priority: 16.10.2014 RU 2014141792
(71) Applicant: Diamondzlite Limited, Tortola (VG); Nazarenko, Anna Borisovna, Moscow 121614 (RU)
(72) Inventor: NAZARENKO, Anna Borisovna, Moscow 121614 (RU)
(74) Representative: Held, Stephan
(86) International application number: PCT/RU2015/000579
(87) International publication number: WO 2016/060587

(57) **Abstract**

The invention relates to medicine and pharmacology, and more particularly to a method for treating and preventing atherosclerosis of major vessels (of the brain, heart and lower limbs) and the aorta using a pharmaceutical agent which contains a therapeutic protein/peptide preparation. The present method comprises administering parenterally or intranasally to a patient a therapeutic preparation in combination with a pharmaceutically acceptable carrier. The therapeutic preparation includes a protein/peptide complex obtained from embryonic brain tissue of cattle and contains negatively charged and neutral proteins and polypeptides with an average molecular mass of from 5 to 200 kDa. The pharmaceutical preparation may additionally contain an immunomodulatory and stimulatory component in the form of hydrolyzed polynucleotides in the form of Na salts of DNA obtained from the milt of sturgeon or salmon. As pharmaceutically acceptable carrier, the preparation contains a diluent, including a saline or buffered saline solution, and possibly various adjuvants. Depending on the severity of the illness (atherosclerosis, site of vessel damage), the preparation is administered subcutaneously, intramuscularly, intravenously, by slow bolus or intranasally in different cycles with the preparation containing 0.01-0.7 mg of protein/peptide complex or protein/peptide/polynucleotide complex in a physiologically acceptable carrier, for example a diluent. The invention provides for reverse cholesterol transport and increases the effectiveness of atherosclerosis treatment and prevention.

## Description

Use of a protein/peptide complex (hereinafter PPC) produced from embryonic nervous tissue or quickly frozen embryonic brain of hoofed livestock, influencing the reverse cholesterol transport from the vessel wall and the activation profile of monocytes of patients with expressed atherosclerosis of major vessels or of patients with susceptibility to cardiovascular diseases, for treatment and prevention of atherosclerosis, and the method for preventing and treating arterial vessels with atherosclerosis and diseases caused by atherosclerosis of major and peripheral vessels of the brain, heart, lower extremities, and aorta in patients.

### (two variants)

### The art

The invention pertains to medicine, in particular to clinical pharmacology, cardiology, therapy, vascular neurology, vascular surgery, and can be used for the treatment and prevention of atherosclerosis of major vessels (brain, heart, lower extremities) and the aorta with the help of a pharmaceutical preparation containing a protein/polypeptide complex produced from embryonic brain tissue of hoofed livestock.

### Prior art

Cardiovascular diseases occupy the first place as a cause of deaths in Russia, USA and Europe, where contribution of this clinical entity into mortality structure amounts to more than 56%, which cannot but influence average life duration in Russia - 58.9 years (for men), while in Japan this indicator is significantly higher - 87 years [1].

The vast majority of cardiovascular diseases is the result of atherosclerosis of major vessels and the aorta. As early as in 1947, N.N. Anichkov demonstrated that cholesterol accumulation in the arterial intima is one of the first signs of atherosclerosis. Cholesterol transport in the organism is performed by lipoproteins. Lipid source in the arterial intima are low-density lipoproteins (hereinafter - LDL). Many researchers (Soran H., 2011; Orso E., 2011) showed that almost any modification (oxidation, deglycosidation, vortexation, proteolysis, lipolysis, etc.) of native LDL leads to their intensive capture both by subendothelial and other cells [2].

The term "atherosclerosis" comes from 2 Greek words: athere (gruel) and sclerosis (hardening). Atherosclerosis is a disease damaging vessel walls, mostly of muscular and myoelastic arteries, and is based on impairments of the fat and protein metabolism, and, primarily, of the cholesterol metabolism, manifested as imbibition of the vessel wall with proteins and lipids, with the development of reactive changes around such as deposits, and rapid growth of connective tissue.

In general, manifestations and various complications of atherosclerosis are rightfully considered to be most frequent reasons of not only physical disability, but also mortality in many countries of the world. Atherosclerosis is most frequently diagnosed among people older than 45-55 years, who keep a sedentary lifestyle and/or abuse alcohol and smoke. It should not be left unmentioned that white-collar workers suffer from atherosclerosis more often than blue-collar workers, and urban citizens are more exposed to this disease than the rural population [3].

The development of cellular biology and biomedicine allows profound research on the impact of various substances on the human organism with the use of models in vitro and ex vivo. Modem research methods demonstrated the possibility to determine many peculiarities of pathobiochemical cascades of human cells' regulatory systems in the metabolism of triglycerides, LDL, cholesterol, allowing the identification of individual peculiarities of some patients [4-6].

Today, drugs from the following groups are used with the predominant impact on certain stages of the cholesterol metabolism:
1. Agents reducing the absorption of cholesterol and bile acids from the intestine (bile acid "sequestrants"):
   - Cholestyramine (Questran, Colestipol);
   - Huar gum (Guarem).
2. Agents influencing the cholesterol transport:
   - Nicotinic acid.
3. Inhibitors of the cholesterol synthesis in the liver:
   - Lovastatin (Mevacor, Mevinacor, Pravastatin, Sinvastin, Fluvastatin);
   - Fibrates (clofibrate, bezafibrate, fenofibrate, gemfibrozil, cyprofibrate).
4. Agents influencing the cholesterol catabolism and removal:
   - Lipostabil.
5. Various hypolipidemic medications (Probucol, polyunsaturated fatty acids, combination of medications).

All specified hypolipidemic drugs are prescribed in case of atherosclerosis of cerebral and coronary vessels, hyperlipoproteinemia among patients with cardiac angina, after myocardial infarction, brain attack, patients with diabetes mellitus [7].

One of the important tasks of modern pharmacology is the development of new medicinal products influencing the metabolism of triglycerides, cholesterol and lipoproteins directly in the atherosclerosis plaque, among patients with already diagnosed disease at all its stages or among patients with a predisposition to cardiovascular diseases. However, cholesterol reduction in blood is not always accompanied by cholesterol reduction in the vessel wall. Therefore, it is especially important to develop new drugs directly influencing cholesterol outflow from the vessel wall and reducing the level of atherogenic potential of blood serum in patients with atherosclerosis.

Blood serum of patients with atherosclerosis, in contrast to blood serum of healthy people, possesses atherogenic properties. "Atherogenic potential" or "atherogenicity" is the ability of blood serum or its components to induce the accumulation of cholesterol esters in the cells of the intima of the human aorta. The intracellular cholesterol accumulation and outflow determines whether cholesterol retention in the vessel wall occurs, that is why cholesterol outflow stimulation is no less important for the reduction of the risk of atherosclerosis development than prevention of intracellular cholesterol accumulation. Cholesterol outflow is a manifestation of atherosclerosis regression on the cellular level. The major ways to influence this process are:
- Activation of the intracellular metabolism of lipids with removal of accumulated lipids from cells;
- Suppression of capture of modified LDL by cells;
- Suppression of the native LDL modification processes;
- Removal of modified LDL from the blood flow.

An integral indicator of the efficiency of various anti-atherosclerotic effects is the reduction of the accumulation rate of intracellular lipids and the reduction of intracellular pool of the cholesterol esters.

At the present time there are no drugs possessing a direct anti-atherosclerotic effect to the full extent. It is known that regular intake of various drugs can influence the process of cholesterol accumulation in the cells of the arterial wall. A range of drugs of different chemical groups can contribute to reduction of the atherogenic potential of the blood serum of patients with atherosclerosis.

The most promising drugs from the whole group of anti-atherosclerotic agents are:
- Inhibitors of squalene synthase and squalene-2,3-epoxidase, suppressing cholesterol biosynthesis without affecting the synthesis of geranyl pyrophosphate, including dolichols and coenzyme Q10;
- Inhibitors of the biosynthesis of isoprenoids;
- Inhibitors of the formation of cholesterol particles, such as specific inhibitors of the microsomal transfer protein; however, such drugs can induce an increase of particles in the cholesterol pool in hepatocytes and contribute to development of a fatty liver or non-alcoholic hepatitis;
- Inhibitors of acyl-cholesterol-acyltransferase - avisimibe preparations ;
- Inhibitors of the intestinal absorption of lipids - compounds like sitostanol/sitosterol as competitive inhibitors of the cholesterol absorption in the intestinal wall;
- Cholesterol absorption inhibitors - preparations with Ezetimibe, which affects the complex Niemann-Pick C1-like protein 1 with annexin, 2-caveolin and sterol transporters, but does not affect the level of vitamin E and does not produce any adverse effects on the gastrointestinal tract;
- Inhibitors of the transport of cystic bile acids - drugs blocking the transport protein of cystic bile acids (IBAT - Inducible/Intestine Bile Acids Transporters), which ensure bile entry to the liver's portal vein, with enzyme induction of the synthesis of bile acids from cholesterol-7-hydroxylase;
- Fibrates (glitazones) - peroxisome proliferation agonist receptors (PPAR) increasing the expression of insulin receptors in muscles and, accordingly, increasing sensitivity to insulin;
- Agonists of liver X-receptors (LXR) - C3-fatty acids reduce triglyceride levels while not influencing the LDL level, since they inhibit the ABC-A1 cholesterol transporter and mitigate the risk of death caused by unexpected cardiopulmonary arrest;
- Inhibitors of cholesteryl ester transfer protein (CETP) - ethanol - inhibits CETP and increases LDL level, which reduces the risk of progression of atherosclerosis,
- Inhibitors of acidified cholesterol forms - the antioxidant probucol inhibits the activity of macrophages through cholesterol receptors;
- Enzymes of high-density lipoproteins - 20-30 proteins, risk factors of coronary syndrome progression, such as hydrolase-thrombocyte activated factor (phospholipase A2) - is an anti-atherosclerotic factor within HDL composition and pro-atherosclerotic factor within LDL composition;
- HDL proteins and peptides are correlated with some pathological phenotypes, such as amyloidosis, nephropathy and atherosclerosis [8].

There are known methods for the treatment of atherosclerosis, which consist of the course prescription of drugs such as linaetholum, nicotinic acid, thyroid hormones, estrogens, essential phospholipids [9]. Specified drugs contribute to the normalization of the metabolism of fats in the organism and to the reduction of cholesterol level in blood.

A common disadvantage of the treatment methods for atherosclerosis using the above-mentioned drugs is a relatively low reduction of the cholesterol concentration in blood serum. Thus, a daily intake of 12-24 g of Cholestyramine leads to a reduction of cholesterol in blood serum by 25%. Upon long-term administration of nicotinic acid in the daily dose of 3 g, it was noted that the cholesterol level in blood serum was lowered by 10% and that of triglycerides by 20%. The use of D-thyroxine in a daily dose of 4-8 mg reduces the concentration of low-density lipoproteins by 20% without significantly changing the concentration of very low-density lipoproteins. At the present time, drugs from polyunsaturated phospholipids of soybeans - Lipostabil (Germany) and Essentiale (Yugoslavia) - are also used as anti-atherosclerotic agents. Methods of their preparation are not described in scientific and technical literature, but there is some indirect evidence to the fact that such drugs contain salts of deoxycholic acid, whose presence negatively affects cellular processes [10].

There is a known method for the prevention and treatment of atherosclerosis by the prescription of carotinoid containing preparations in the daily dose of 5-10 mg/kg of the body weight, depending on the character of the prescription, which ensures an effective level of carotenoids content in blood serum. For the increase of dosage accuracy, it is possible to take into account the carotinoid level in each certain case in the patient's blood serum, - when this or that dose of drug is prescribed for such patient. In particular, the lycopene level in blood serum shall not be below 700-800 nM.

Maintenance of an effective carotinoid concentration in blood serum shall be ensured with account on the fact that carotenoids are moderately rapidly metabolized and eliminated from the human and animal organism. For example, in case of a single peroral administration of lycopene to rabbits, its maximum concentration in blood serum will be observed after 8 hours, and it will return to the initial level after 24 hours.

Individual carotenoids extracted from different sources or aggregated fractions of carotenoids, for example the carotinoid crocetin, can serve in carotinoid preparations.

Carotenoids of various degrees of purity can be used in the form of powders or oily solutions, or dispersions, which increases their absorption in the gastrointestinal tract.

As pharmaceutical forms, powders, pills, suppositories, oily solutions or emulsions can be used.

The type of administration of carotenoids does not have any decisive significance, but it is more convenient to use them orally or rectally.

Other administration routes (intramuscularly, intravenously) are also feasible, but they seem not to have any specific advantages and practical importance in therapeutic practice, though in some cases they can be used, for example in experimental works.

Regularity of carotenoid intake is not specifically important as such. It is most important to observe the recommended daily dose and to maintain an effective level of drug concentration in the blood serum.

It was also found that upon feeding rabbits for 28 days with food containing 1% cholesterol, elasticity of endothelial vessels was preserved, if 0.6 g of beta-carotene were simultaneously added to each 1 kg of food, and no impact of beta-carotene on LDL resistance to oxidation was detected.

The addition of 125-500 mg of beta-carotene per 1 kg of food together with cholesterol-rich food for hypersensitive rats for 44 days resulted in a dose-dependent reduction of cholesterol and triglyceride levels in blood serum.

As for guinea pigs who received daily 0.2% cholesterol and 15-30 mg of beta-carotene for 12 weeks, no verifiable differences in the cholesterol and triglyceride levels in blood serum were found, but with a 50 mg dose of beta-carotene/100 g of food for 12 weeks, a significant reduction of sizes of sclerotic clots in aorta was noted.

Upon research on the impact of beta-carotene on the level of lipoproteins in blood serum of smoking people, thus, referring to the risk group, no visible effect was identified after a 14-week intake of beta-carotene in the dose of 20 mg/day, though the beta-carotene level in blood serum increased by 15 times [11].

Therefore, there are serious reasons to suggest that carotenoids (beta-carotene was mostly researched among them) are the effective anti-atherosclerotic agents, since in most cases they eliminate major biochemical impairments caused by atherosclerosis.

One disadvantage of the known methods for the prevention and treatment of atherosclerosis using carotenoid drugs is the insufficient reproducibility of results, which is, in particular, evidenced by the above-provided literature data, which is not consistent enough. One of the main difficulties in comparing the efficiency of experimental atherosclerosis treatments is the use of different principles for the dosage of the carotenoid drugs, which is mostly expressed by the ratio between the dose of the preparation and the unit of the food weight, and in other cases in the form of some random fixed values of prescribed doses. An ineffective use of carotenoids sometimes found can be explained, in the applicant's opinion, by too low doses of the drug.

There are also known methods for the production and use of polypeptide drugs normalizing blood vessels' functions, used among patients with cerebral atherosclerosis.

In particular, SU No 1227198, 1986 describes a complex of polypeptides extracted from blood vessels of animals, reducing the cholesterol content in the blood. The complex is produced by extraction of finely divided blood vessels of animals by a 3% aqueous solution of acetic acid, subsequent centrifugation, treatment of the supernatant at the temperature from -3°C to -5°C, precipitate dissolution, filtration, and lyophilisation.

The disadvantage of this complex limiting its use is the extraction of a large amount of non-peptide substances being impurities and not serving as biologically active substances.

RU No 2301072, 2007 describes a method for producing a drug produced on the basis of biologically active substances obtained from the animal organs (from blood vessels). The produced peptide complex contains low-molecular fractions comprising 70-90% of peptides with the molecular mass from 72 to 678 kDa, with a peptide concentration in aqueous solution of 2.5-2.9 mg/ml. The use of this drug for treating patients with cerebral atherosclerosis was analyzed. It was found that the use of this drug contributed to the improvement of the general well-being. Total cholesterol prior to treatment was 8.4 ± 0.6 mmol/L, after treatment 6.2 ± 0.2 mmol/L, lipoproteins content prior to treatment was 1.3 ± 0.07 mmol/L, after treatment 0.86 ± 0.8 mmol/L.

These patent sources lack any data about the influence of such peptide drugs on metabolic processes directly in the atherosclerotic clot and subendothelial vessel layer.

Thus, one of the main risk factors significant upon progression of these diseases is the high level of lipids in blood plasma, in particular, a high cholesterol level. A lot of efforts were made to use an agent reducing the cholesterol level for the prevention and treatment of such diseases, and a lot of compounds were found, which to a greater or a lesser extent possess such an effect. For example, one of such compounds, which is well known and successfully applied, is pravastatin, which is the drug regulating lipids and the inhibitor of 3-hydroxy-3-methyl-glutaryl-coenzyme A reductase (in this document it is specified as "Inhibitor of HMG-CoA reductase), and, it is supposed to act at the stage of cholesterol biosynthesis determining its rate. It was reported that for rabbits receiving pravastatin it is possible to prevent coronary atherosclerosis and xanthoma, however, its effectiveness is not sufficient (Biochimica at Biophysica Acta, 960, 294-302 (1988)). Experiments on control over coronary atherosclerosis and xanthoma were carried out with the use of the combination of two drugs regulating lipids, pravastatin and cholestyramine, which are well known as drugs reducing lipoprotein levels, however the effectiveness of such a combination also remains insufficient (Atherosclerosis, 83, 69-80, 1 (1990)).

In the Japanese patent application 7-41423, it is suggested that a specific class of agents improving resistance to insulin, for example troglitazone, can be effective for the treatment and prevention of arteriosclerosis, but even in that case the effectiveness of such compounds is not satisfactory enough.

At the present time, for treating and prevention of atherosclerosis, non-pharmacological means of eliminating or reducing risk factors, and drugs, for example various hypolipidemic agents (cholestyramine, polisponin, linaetholum, heparine, chondroitin sulphate, etc.) (Mashkovskiy M.D. Medicinal agents, part 1 and 2, M. Medicine, 1987, v. 2, p. 89, 92, 73, 156) are used.

None of the existing hypolipidemic drugs is universal, and almost all of them have adverse effects. The search for new anti-sclerotic agents is being carried out among various classes of chemical compounds:
- Urea and thiourea derivatives (USA patents No.No. 4397868, 4387105, 4623662);
- Aminobenzoic acid derivatives (application of the European Patent Office No. 0170361, accepted applications of Japan 59-24135, 63-26729);
- Benzothiazines (application of the European Patent Office No. 0174458);
- Indole derivatives (Great Britain application No. 2190587);
- Hexahydronaphthalene derivatives (USA patent No. 4444784).

Common drugs applied for atherosclerosis treatment are clofibrate (Miscleron, Lipomid, Atromid), and isobutyrate (Mashkovskiy M.D. Medicinal agents, part 1 and 2, M. Medicine, 1987, v. 2, p. 90).

These reduce the content of lipoproteins rich in cholesterol and triglycerides in blood. They are used for atherosclerosis with a high content of these substances in blood.

However, treatment with clofibrate is not effective enough, and it does not prevent the ischemic heart disease and induces a range of adverse effects. It is capable of inducing intrahepatic cholestasis, accompanied by the formation of stones in the gall bladder and the biliary tract.

From RU No. 2243767 priority 10.01.2005, another method for the treatment of atherosclerosis is known. This method is implemented on the background of drug therapy by agents from the group of statins, teveten, polyoxidonium, and performance of plasmapheresis sessions with the removal of 800 ml of plasma 2 times per week No. 5 by means of additional administration to the patient of Imunofan 0.005% - 1.0 intramuscularly on day No. 10 and Fluimucil 300 mg intravenously daily No. 5-10, and total duration of a course being 2 months. The method ensures the modulation of the functional activity of leukocytes, including, indirectly through the change of the cytokine profile and, as a result, the disintegration of protein-lipid complexes participating in the formation of the atherosclerotic plaque.

It is known that the majority of cardiovascular diseases result from atherosclerosis of major vessels and the aorta (Pranavchand R., 2013). Back in 1947, N.N. Anichkov demonstrated that cholesterol accumulation in the intima of arteries is one of the first signs of atherosclerosis (Anichkov N.N., 1947; Cianciola N.L., 2011). Cholesterol transport in the organism is performed by lipoproteins. Lipid source in the arterial intima are low-density lipoproteins (hereinafter - LDL) (Vicovic I., 2006; Alaupovic, 1971 P.; Coocson F.B., 1971). Many researchers (Soran H., 2011; Orso E., 2011) showed that almost any modification (oxidation, deglycosidation, vortexation, proteolysis, lipolysis, etc.) of native LDL leads to their intensive capture both by subendothelial and other cells.

The development of cellular biology and biomedicine allows profound research on the impact of various substances on the human organism with the use of in vitro and ex vivo models. Modem research methods demonstrated the possibility to determine many peculiarities of pathobiochemical cascades of human cells' regulatory systems in the metabolism of triglycerides, LDL, cholesterol, allowing the identification of individual peculiarities of some patients.

One important task of modern pharmacology is the development of new medicinal products influencing the metabolism of triglycerides, cholesterol, and lipoproteins among patients with a predisposition to cardiovascular diseases or among patients with an already diagnosed disease at all its stages.

In recent years, a cell model was developed (cell culture test), which allows the estimation of cholesterol outflow from the vessel wall as an integral indicator of reverse cholesterol transport. It was found that cholesterol reduction in blood is not always accompanied by cholesterol reduction in the vessel wall. The model is used in vitro for the assessment of the effectiveness of hypolipidemic drugs of different classes of pharmacological substances on reducing the level of the atherogenic potential of blood serum of patients with atherosclerosis.

Blood serum of healthy people, in contrast to blood serum of people with atherosclerosis, does not possess any atherogenic properties.

The outflow of cholesterol, which is part of the mechanism of reverse cholesterol transport in the organism, is the reverse side of cholesterol accumulation in the arterial cells. The accumulation of intracellular cholesterol and its outflow determine whether cholesterol retention in the vessel wall occurs. The stimulation of cholesterol outflow is no less important for eliminating the risk to develop atherosclerosis than the prevention of intracellular cholesterol accumulation. On the cellular level, cholesterol outflow is a manifestation of atherosclerosis regression. There is a number of possibilities to influence this process, in particular: removal of modified LDL from the blood flow, suppression of the native LDL modification processes, activation of the intracellular metabolism of lipids, elimination of the capture of modified LDL by cells, elimination of accumulated lipids from the cells. An integral indicator of the efficiency of various anti-atherosclerotic effects is the decrease of the rate of accumulation of intracellular lipids and the decrease of the intracellular pool of cholesterol esters.

At the present time there are no drugs possessing a direct anti-atherosclerotic effect to the full extent. It is known that regular intake of various drugs can influence the processes of cholesterol accumulation in the cells of the arterial wall. A range of drugs of different chemical groups can contribute to reduction of the atherogenic potential of the blood serum of patients with atherosclerosis.

A cell culture test is regarded as the most appropriate and suitable method for modeling early stages of atherogenesis on the cellular level. The use of an "ex vivo" model for the assessment of the anti-atherogenic potential of drugs made it possible to carry out series of tests required for streaming drug screening.

### Invention disclosure

Technical task of the claimed invention is the increase of effectiveness of the treatment and prevention of atherosclerosis of arterial vessels, diseases induced by atherosclerosis of major and peripheral vessels of the brain, heart, lower extremities, and aorta, and creation of such method for treating and prevention with the use of a cellular medication ensuring cholesterol outflow from the vessel wall.

The stated technical task and obtained technical result are achieved by the claimed inventions group, comprising theuse of a protein/polypeptide complex or a protein/peptide-polynucleotide complex for the treatment and prevention of atherosclerosis and the method for prevention and treating of atherosclerosis of arterial vessels and diseases caused by atherosclerosis of major and peripheral vessels of the brain, heart, lower extremities, and aorta in patients.

Thus, one of the subject matters of the claimed invention is the use of a protein/peptide complex (hereinafter - PPC) produced from embryonic nervous tissue of the hoofed livestock or from quickly-frozen embryonic brain of the hoofed livestock of gestation age from the middle of first third till the middle of the last third of pregnancy, comprising negatively charged, neutral proteins and polypeptides, with the molecular weights ranging from 0.5 to 200 kDa, therewith at least 70% of the total protein amount having molecular weights ranging from 20 to 180 kDa, having a specific set of bands at denaturing gel electrophoresis ("SDS-Page") in 5% polyacrylamide gel compared to the standard set of marker proteins having molecular weights from 0.5 kDa to 200 kDa, or in their combination with polynucleotides in the form of sodium salts of DNA extracted from milt of sturgeons and salmons families, in the form of protein/peptide-polynucleotide complex for the prevention and treatment of atherosclerosis of arterial vessels and diseases caused by atherosclerosis of major and peripheral vessels of the brain, heart, lower extremities and aorta, in patients, administered parenterally or intranasally in therapeutically effective doses, and regulating the metabolism of cholesterol, cholesterol esters, triglycerides, and phospholipids in subendothelial connective-tissue and smooth-muscle cells of arterial vessels.

Protein/polypeptide complex (hereinafter - PPC) and protein/peptide-polynucleotide complex (hereinafter - PPPC), the use of which is one of the subject matters of this invention, are produced by methods known from the patent RU No. 2445106, 20.03.2012, RU 2428196, RU No. 2460736, RU No. 2477637, RU No. 2485133, and RU No. 2485132.

The stated technical task and obtained technical result are also achieved by the method for prevention and treatment of atherosclerosis of arterial vessels and diseases caused by atherosclerosis of major and peripheral vessels of the brain, heart, lower extremities, and aorta, in patients, comprising the parenteral or intranasal administration to patients of a drug comprising a protein/peptide complex (hereinafter - PPC) in combination with the pharmaceutically acceptable carrier in therapeutically effective daily or course doses, as a drug regulating the metabolism of cholesterol, cholesterol esters, triglycerides, and phospholipids in subendothelial connective-tissue and smooth-muscle cells of arterial vessels; wherein, the drug comprises a protein/peptide complex produced from embryonic nervous tissues from the brain of the hoofed livestock, and containing negatively charged, neutral proteins and polypeptides with the molecular weight from 5 to 200 kDa, and a fraction content of at least 80% with an average molecular mass from 10 to 120 kDa, a total protein content of 0.05 - 4.2 mg/ml, with the maximum absorption peak of a UV spectrum of the solution at the wavelength 280±5nm, with a peak present at the wavelength 274-284 nm in the UV-visible spectrum, and the presence of specific bands within pl range from 4.2 to 8.4 at isoelectric focusing in 5% polyacrylamide gel.

The stated technical task and obtained technical result are also achieved by the method for prevention and treatment of atherosclerosis of arterial vessels and diseases caused by atherosclerosis of major and peripheral vessels of the brain, heart, lower extremities, and aorta, in patients, comprising the parenteral or intranasal administration to patients of a drug comprising a protein/peptide complex (hereinafter - PPC) in combination with the pharmaceutically acceptable carrier in therapeutically effective daily or course doses, as a pharmaceutical preparation regulating the metabolism of cholesterol, cholesterol esters, triglycerides, and phospholipids in subendothelial connective-tissue and smooth-muscle cells of arterial vessels; wherein, the preparation contains a protein/peptide complex produced from nerve tissues of hoofed farm embryos of gestation stage from the middle of the first third to the middle of the last third of pregnancy, comprising tissue-specific negatively charged, neutral proteins and polypeptides classified as growth and differentiation factors, signaling molecules determining its biological and pharmacological activity, with molecular weights ranging from 0.5 to 200 kDa, therewith at least 70% of the total protein amount having molecular weights ranging from 20 to 160 kDa, with a total protein content of 0.05-4.2 mg/ml, having a specific set of bands at denaturing gel electrophoresis ("SDS-Page") in 5% polyacrylamide gel compared to the standard set of marker proteins having molecular weights ranging from 1 kDa to 250 kDa, an isoelectric point from 4.2 to 8.4, and an absorption peak at a wavelength of 215±5 nm in the ultraviolet spectrum recorded at wavelengths ranging from 200 to 500 nm.

Therapeutic drug used for the prevention and treatment of atherosclerosis of arterial vessels and diseases caused by atherosclerosis of major and peripheral vessels of the brain, heart, lower extremities, and aorta, in patients, containing a protein/peptide complex (mentioned above) and the pharmaceutically acceptable carrier can contain hydrolyzed polynucleotides in the form of sodium salts of DNA, produced from the milts of sturgeons and salmons families, representing a medium- and low-molecular polynucleotide fraction in the form of sodium salt of desoxyribonucleic acid, demonstrating (in complex with the protein/peptide fraction) specific immunoregulatory activity in at least equal concentration with the protein/polypeptide complex, and pharmaceutically acceptable diluent; wherein, medium- and low-molecular polynucleotide fractions in the form of DNA sodium salts are characterized by a molecular weight from 12 to 660 kDA (18-1000 base pairs), having a maximum absorption at the UV wavelength 260 ± 2 nm and the minimum one at 230±2 nm in the range of wave lengths from 190 to 325 nm with a ratio of B260/280 from 1.6 to 2.0, the presence of characteristic specific bands in 1.85-agarose gel upon ethidium bromide coloring, in contrast to the standard set of markers with the range of molecular masses from 75 to 7000 base pairs, and blue staining at determination of qualitative reaction to ribose, as well as with addition of protein upon determination of its content by Lawry assay as not higher than 0.18%.

As hydrolyzed polynucleotides together with the protein/peptide complex in the claimed invention pertaining to the treatment and prevention of atherosclerosis of arterial vessels and diseases caused by atherosclerosis of major and peripheral vessels of the brain, heart, lower extremities, and aorta, in patients, hydrolyzed polynucleotides in the form of DNA sodium salts can be used (in the pharmaceutical preparation), which are produced from the milts of sturgeon and salmon families, with the chain length of the polynucleotides from approximately 1000 to 7000 nucleotides as the component immune-modulating and stabilizing proteins and polypeptides in equal or sufficient therapeutic daily and course dose. Their concentration in this protein/peptide-polynucleotide complex (hereinafter - PPPC) is approximately 0. 1-3.0 mg/ml (milligram per milliliter) with the total protein content in pharmaceutical preparation 0.05-4.2 mg/ml.

The concentration of sodium salts of DNA in the protein/peptide-polynucleotide complex amounts to approximately 0.05-3.0 mg/ml with the total protein content in the pharmaceutical preparation equal to 0.05-4.2 mg/ml.

A pharmaceutically acceptable diluent, the diluent containing buffer solution and, possibly, excipients (surfactants, reversible inhibitors of proteases and deoxyribonucleases) is used.

Please, find below necessary and possible therapeutically effective doses of a pharmaceutical preparation containing the protein/polypeptide complexes (PPC) specified above, or a pharmaceutical preparation containing PPC and hydrolyzed polynucleotides in the form of DNA sodium salts, produced from the milts of sturgeon and salmon families (hereinafter - PPPC pharmaceutical preparation).

According to the claimed method, the following (certain) treatment regimens are implemented:
- in particular, during one procedure a pharmaceutical preparation can be administered to the patient, containing from 0.05 to 1.0 mg of PPC in 1.0 - 5.0 ml of pharmaceutically acceptable carrier-diluent in the form of 8-10 injections, 1-2 times daily, wherein the course 8-14 days; or
- in particular, during one procedure a pharmaceutical preparation can be administered to the patient, containing from 0.05 to 1.0 mg of PPPC in 1.0 - 5.0 ml of pharmaceutically acceptable carrier-diluent in the form of 8-20 injections, 1-2 times daily, wherein the course is 8-14 days, and the ratio between PPC and polynucleotides from the fish milt can be 0.1-2 : 0.5-2 respectively; or
- in particular, during one procedure a preparation is administered intravenously by drip or slow bolus to the patient, containing from 0.025 to 0.7 mg of PPC in 5.0 - 500.0 ml of pharmaceutically acceptable diluent, 1-2 times daily, wherein the course is 5-12 days; or
- in particular, during one procedure a preparation is administered intravenously by drip or slow bolus to the patient, containing from 0.025 to 0.7 mg of PPPC in 5.0 - 500.0 ml of pharmaceutically acceptable diluent, 1-2 times daily, wherein the course is 5-12 days; or

In accordance with the claimed invention, a course of treatment can be performed by subcutaneous administration of PPC once per 4 months in the dose of 0.05-0.1 mg in 1.0-5.0 ml of pharmaceutically acceptable carrier (diluent), and in addition 1 ampoule (of one corresponding injection) is administered for 10 days, and two 14-day courses are performed by intranasal administration of two drops in each nasal duct in the morning with an interval of 2.0-2.5 weeks between the courses.

In the claimed invention, the dosage of the drug expressed in "mg" mostly refers to subcutaneous injections. And, in particular, the course dose for injection course amounts to 10-14 injections with repeat in 7-10 days. Quantitative representation of "µg" mostly refers to dosages for intranasal spray, for example, one injection - 100 µl or "µg" (if density of the used solution is taken as a unit). Nasal mucosa, as a rule, is not able to accept more than 2-3 injections in each nasal duct, therefore the maximum dose for intranasal administration by volume amounts to no more than 400 µl or 400 µg of the used solution (in particular, 0.01% solution).

Besides, according to the claimed methods, the following (certain) treatment regimens are implemented:
- in particular, during one procedure a preparation is administered intranasally, containing from 0.001 to 0.1 mg of PPC in 0.05 - 1.2 of pharmaceutically acceptable carrier-diluent in the form of 2-3 injections or 2-4 drops in each nasal duct, 1-2 times daily, wherein the course is 10-14 days; or
- in particular, during one procedure preparation is administered intranasally, containing from 0.001 to 0.1 mg of PPPC in 0.05 - 1.2 ml of pharmaceutically acceptable diluent in the form of 2-3 injections or 2-4 drops in each nasal duct, 1-2 times daily, wherein the course is 10-14 days.

In the claimed method, drugs containing PPC and PPPC are administered together with other anti-atherosclerotic drugs selected from the following groups:
- bile acid sequestrants; inhibitors of cholesterol synthesis in the liver (statins);
- substances influencing cholesterol absorption, catabolism, and clearance;
- hypolipidemic drugs,
in therapeutically effective and required doses.

In the claimed method, systematic course administration of drugs based on PPC or PPPC is performed in 4-12 courses per year, with an interval on 10-56 days between the courses, depending on the intensity of atherosclerotic process, its localization, the existence of any complications, and their consequences.

It is reasonable upon preventive treatment of atherosclerosis, i.e. for prevention of atherosclerosis of vessels, to perform a systematic course administration of drugs based on PPC and PPPC, in the form of 2-8 courses per year, with intervals of 1-4 months between the courses, depending on the aggregation risk factors and genetic predisposition of the patient.

In accordance with the claimed method in case of preventive treatment, i.e. for prevention of atherosclerosis of vessels, it is preferable to perform a systematic course administration of drugs based on PPC and PPPC, in the form of 2-6 courses per year, with intervals of 2-6 months between the courses, depending on the aggregation risk factors and genetic predisposition of the patient, and it shall be combined with the use of other antiatherosclerotic drugs selected from the group containing:
- bile acid sequestrants;
- inhibitors of cholesterol synthesis in the liver (statins);
- substances influencing cholesterol absorption, catabolism, and clearance;
- hypolipidemic drugs,
in therapeutically effective and required doses.

As a pharmaceutically acceptable carrier of the drug based on protein/peptide complex (PPC) or protein/peptide-polynucleotide complex (PPPC), traditional pharmaceutically acceptable carriers and diluents are used, for example saline (sodium chloride) solutions or phosphate buffer solutions and, possibly, different auxiliary additives (substances), in particular, emulsifiers, suspending substances, preservatives, solvents with high boiling point, distilled water.

The term "therapeutically effective amount" used in the claimed invention ("therapeutically effective required doses") means the amount of substance effective for prevention and treatment of atherosclerosis.

Upon production of a pharmaceutical or medicinal preparation containing PPC and PPPC, for example, phosphate buffer solution is mixed (1.5 M NaCl, 0.1 M Na-phosphate), then distilled water is added, hydrolyzed polynucleotides are added (in the form of sodium salts of DNA produced from the milts of sturgeon and salmon families) and at last the protein/peptide complex is added. This combination is thoroughly mixed. The preparation volume is filled with distilled water up to 100 ml and thoroughly mixed. The solution is sterilized.

According to the claimed method, the pharmaceutical or medicinal preparation based on PPC or PPPC (combined with a physiologically acceptable carrier, in particular, a diluent) can additionally contain other different known antiatherosclerotic substances selected from the group, comprising bile acid sequestrants [cholestyramine (Questran, Colestipol, huar gum (izarem)]; inhibitors of the cholesterol synthesis in the liver [statins, for example lovastatin (Mevacor, Mevinacor, Pravastatin, Sinvastin, fizvostatin)], fibrates (clofibrate, bezafibrate, fenofibrate, gemfibrozil, cyprofibrate); additives influencing cholesterol absorption, catabolism, and clearance (nicotinic acid, lipostabil), hypolipidemic drugs (probucol, polyunsaturated fatty acids, combined agents), used in therapeutically effective doses.

Methods for producing the protein/peptide complex (PPC) used in the claimed method for the treatment and prevention of atherosclerosis are described, in particular, in RU 2445106, 20.03.2012, and RU 2485132, 20.06.2013.

Polynucleotides (in the form of sodium salts of polynucleotides) used in the claimed method (in one of its embodiment variants) are produced by methods similar to the methods described, for example, in the prior art, in particular, patents RU 2072855, 10.02.1997, RU 2034554, 10.05.1995, RU 915446, RU 2078581, i.e. under known techniques comprising homogenization of raw materials in a buffer solution, centrifugation, filtration, ultra-filtration, deproteinization, etc. The molecular mass of the obtained sodium salt of DNA can be regulated by different methods, for example, by varying the amount of added EDTA (ethylenediaminotetraacetic acid).

In particular, hydrolyzed polynucleotides in the form of sodium salts of DNA are used, which are obtained from the milts of sturgeon and salmon families by different known methods, for example, the method from RU No. 2485132, 20.06.2013.

For research on the impact of the original PPC and PPPC with the conditional name "Cellex" (hereinafter - Cellex, as a modifier and activator of biological reactions) on the level of reduction of the atherogenic potential of blood serum of patients having atherosclerosis according to the known method [5], a primary culture of subendothelial cells of the human's aorta was obtained from various sections of the intima of the thoracic aorta of men and women aged 40-65 years within 1.5-3 hours after sudden death, which differed by the degree of atherosclerotic disease, then it was treated by collagenase, and the cells were cultured. A 7-10-day primary cell culture was used - a mixed cell population mostly consisting of the typical and modified smooth muscle cells.

A primary culture of cells, extracted from the intima of aorta's sections not damaged by atherosclerosis, was used for the reproduction of the processes of atherogenesis at the cellular level and the assessment of antiatherogenic properties of the analyzed substances. Antiatherogenic action was deemed to be the effects impeding major manifestations of atherogenesis at the cellular level, and, first of all, accumulation of intracellular cholesterol.

A primary culture of cells, extracted from the intima of aorta's sections damaged by atherosclerosis, was used for the assessment of antiatherosclerotic properties of Cellex. Antiatherosclerotic actions were deemed to be the effects manifested in reduction of intracellular cholesterol content compared to the initial level.

In the clinical studies upon serial measurements of atherogenic properties of the blood serum (its ability to induce accumulation of intracellular cholesterol), the culture of blood monocytes-macrophages of healthy volunteers was used, which was cultured until their maturation into macrophages [14].

Blood serums of the patients were added into cell cultures in the final concentration of 40% (upon use of the primary culture of subendothelial cells of non-damaged human's aorta intima) or 10% (upon use of the culture of human blood monocytes-macrophages). As a means of control, the cells which were used were further cultured in the medium 199 containing antibiotics and 10% veal embryo's serum, which contains necessary cell growth factors but does not influence the intracellular lipid content.

Determination of the content of intracellular cholesterol, intracellular lipids and cellular protein was made according to the standard methods of the pharmacopoeia.

### Calculation of the atherogenic effect (assessment of blood serum atherogenicity)

After determination of total cholesterol and protein content, the ratio cholesterol/protein was calculated in each probe. The unique content of the total cholesterol in cells extracted from non-damaged sections of the aorta's intima varied within the range of 20-50 µg/mg of cellular protein. The specific content of the total cholesterol in human blood monocytes-macrophages varied within 8-20 µg/mg of cellular protein. The average specific cholesterol content in control cells of this series was accepted as 100%, and variation did not exceed 8%. The atherogenic effect of the studied serums was determined according to the cholesterol content in the test cultures and expressed in percents from the intracellular cholesterol content in the control.

Patient's blood serum inducing a statistically reliable accumulation of intracellular cholesterol was considered as atherogenic.

Assessment of the cholesterol outflow (antiatherosclerotic effect) in the models in vitro and ex vivo.

Intracellular cholesterol content in control cells incubated without Cellex addition was accepted as 100%.

The effect on the cholesterol outflow (antiatherosclerotic effect) of the studied substances was determined as their ability to reduce statistically reliably the initially high content of intracellular cholesterol.

For the ex vivo model, blood was taken from volunteers immediately prior to Cellex intake and after the corresponding intervals after the intranasal intake (after 2, 4, and 6 hours for screening studies and assessment of the short-term effect, as well as after 4, 8, 12, and 24 hours for the assessment of duration of the effect).

### Determination of the synthesis of collagen

Cells extracted from normal or damaged sections of the intima of human's aorta were incubated for 24 hours in the presence of 5 µCi/ml [3H]-proline. Upon completion of incubation, inclusion of marked proline into the culture medium fraction treated by collagenase was determined by the method of Peterkofsky and Diegelmann.

**Table 1. Influence of lipostabil, lovastatin and cellex on the cholesterol content in atherosclerotic cells of the intima of human's aorta in vitro.**

| Drug | Content of intracellular cholesterol (µg/mg) |
|---|---|
| Control | 105 ± 11 |
| Lipostabil | 82 ± 3 * |
| Lovastatin | 110 ± 10 |
| C ellex ** | 76 ± 5 * |

| | |
|---|---|
| Notes: The effect of substances on the intracellular cholesterol content was studied in the concentration 10-6 M, Cellex - 1.0 x 10-6 mg/ml. These data represent average values obtained in three independent experiments; * - significant decrease of intracellular cholesterol content, p < 0.05; ** code name of the drug containing the protein/peptide complex (PPC) described in the application. | |

Table 2 provides data of a complex assessment of the effect of the Cellex drug on atherosclerotic indicators of cultured cells in contrast to the known drug Verapamil, which belongs to the class of calcium antagonists. It is known that Verapamil possesses anti-atherosclerotic effects, inducing atherosclerosis regression among patients with documented atherosclerotic diseases of the vessels. In the primary culture of human aorta's intimal cells, Verapamil dose-dependently reduced total cholesterol content, i.e. contributed to reverse cholesterol transport. In the absence of Verapamil, cholesterol outflow from the cells was not observed. Along with that, free (unesterified) cholesterol did not change. This proves that cholesterol reduction in cells occurs exclusively by means of reduction of cholesterol esters. Along with the reduction of the total cholesterol, reduction of the content of two other essential classes of intracellular lipids, phospholipids and triglycerides was observed. Therefore, reverse cholesterol transport from atherosclerotic cells is accompanied by the reduction of intracellular content of other essential classes of lipids, which shall contribute to the reduction of excessive fat in cells with atherosclerotic damage. Such regression of cellular lipidosis can explain the mechanism of the antiatherosclerotic action of Verapamil.

**Table 2. Antiatherosclerotic effects of Verapamil and Cellex in the in vitro model.**

| Studied parameter | Control (µg/105) | Vera pamil content (M) | Vera pamil effect (% of control) | Celle x content (mg/ml) | Celle x effect (% of control) |
|---|---|---|---|---|---|
| Total cholesterol | 57.4 ± 1.2 | 10-7 | 104 ± 5 | 1.0 x 10-7 | 92 ± 12 |
| | - | 10-6 | 82 ± 8 * | 1.0 x 10-6 | 79 ± 10 * |
| | - | 10-5 | 61 ± 4 ** | 1.0 x 10-5 | 68 ± 8 * |
| Free cholesterol | 46.8 ± 5.3 | 5 x 10-5 | 90 ± 8 | 1.0 x 10-6 | 98 ± 10 |
| Phosph olipids | 71.4 ± 10.9 | 5 x 10-5 | 61 ± 8 * | 1.0 x 10-6 | 69 ± 12 * |
| Triglyc erides | 12.1 ± 1.7 | 5 x 10-5 | 76 ± 2 * | 1.0 x 10-6 | 82 ± 8 * |
| Cholest erol esters | 30.8 ± 1.6 | 5 x 10-5 | | 1.0 x 10-6 | 68 ± 6 ** |
| Inclusio n of [3H] thymidine | - | 10-7 | 86 ± 7 | 1.0 x 10-7 | 92 ± 11 |
| | - | 10-6 | 50 ± 2 * | 1.0 x 10-6 | 64 ± 8 ** |
| | - | 10-5 | 34 ± 2 * | 1.0 x 10-5 | 43 ± 8 ** |
| | - | 10-4 | 20 ± 2 * | 1.0 x 10-7 | 16 ± 4 *** |
| Synthes is of collage nA (dpm/1 03) | 2500 ± 102 | 5 x 10-5 | 74 ± 5 * | 1.0 x 10-6 | 80 ± 6 * |

| | | | | | |
|---|---|---|---|---|---|
| Notes: Significant differences from control: * - p < 0.05; ** - p < 0.01; *** - p < 0.001. | | | | | |

Cellex, as well as Verapamil, induced a dose-dependent significant suppression of proliferative activity of the cultured atherosclerotic cells (Table 2), reducing the levels of the total and free cholesterol, as well as concentrations of triglycerides and esterified cholesterol in contrast to Verapamil. Besides, Cellex, as well as Verapamil, suppressed the collagen synthesis in the primary culture of cells extracted from the atherosclerotic lesion (Table 2).

Thus, Cellex, as well as Verapamil, not only contribute to the cholesterol outflow from atherosclerotic cells, but also possess other anti-atherosclerotic properties at the level of vessel wall cells, while suppressing proliferative and synthetic activity, i.e. reduce or suppress all major manifestations of atherosclerosis at the cellular level - lipidosis, proliferation, and fibrosis.

However, Cellex, in contrast to Verapamil, is not a calcium channel blocker, it does not influence the arterial pressure or cardiac rhythm, and with a comparable antiatherogenic effect (Cellex has a higher one), it is the medication of choice for the long-term use.

### Preferred embodiments

Examples illustrating the invention without limiting it are presented below.

### Example 1.

Patient, 54 years old, was admitted to the vascular neurology department with complaints about speech disorder, diffuse headache, numbness of the left body side, muscle weakness in the left forearm and hand. Disease onset is acute, the patient associates it with having a hot bath.

Anamnesis: Ischemic-type disorder of cerebral circulation 8 months ago, hospitalization with similar symptoms in a neurological department, where ultrasound examination verified intravasal stenosis up to 52% (atherosclerotic plaque?) of the right internal carotid artery at the distance of 2 cm from its origin from the common carotid artery. On the background of performed antiaggregant, neuroprotective, and antioxidant therapy, complete regress of neurologic deficit was noted within 21 days. The patient was discharged for neurologist's supervision according to the place of residence. Heavy smoker for 17 years, smokes 2 packs of cigarettes per day. Chronic bronchitis of a smoker.

Accompanying diseases: arterial hypertension with smooth progression, adapted to AP - 140/90 mm Hg, regularly takes Enap - 2.5 mg x 2 times/day for 1.5 months - Thrombo ASS - 100 mg before night. Lumbar osteochondrosis with regular exacerbations 2-3 times per year in the form of lumbodynia and ischias on the left side. MRI helped to diagnose paramedian hernia of intervertebral disks L4-L5 and 1_5-Si, 8 and 12 mm respectively with lateralization to the left.

Patient's condition upon admission was close to moderately severe, the patient was conscious, accurately oriented, the contact was complicated due to apparent speech disorder, understood speech addressed to him, executed simple instructions. Somatic status - breath 18 per minute, slightly harsh with single sonorous bronchial bubbling rales;

Unpronounced tachycardia up to 96 beats/minute, arterial hypertension - 155/95 mm Hg.

Neurological status: cerebral and meningeal symptoms are absent. Cranial nerves - slight central paresis VII, XII cranial nerve on the left, reduction of the left pharyngeal reflex, apparent dysarthia. Left-sided hemiparesis with muscular tone reduction and range of motions up to 3 scores in the left hand. Anisoreflexia S>D, Babinsky syndrome on the left. Left-sided hemihypesthesia. Coordination and pelvic disorders are not detected.

Diagnosis: Repeated ischemic stroke in the right medial cerebral artery circulation. Discirculatory encephalopathy of degree 2-3 of vascular genesis. Cerebral atherosclerosis with intravasal stenosis of the right internal carotid artery. Arterial hypertension with smooth progression, decompensation. Chronic bronchitis.

During ultrasound examination of major and intracranial cerebral vessels, intravasal stenosis up to 62% (atherosclerotic plaque) of the right internal carotid artery is detected at the distance of 1.8 cm from its origin from the common right carotid artery. MRI in vascular mode confirms apparent thickening of the vessel wall of the right internal carotid artery with stenosis up to 60%; a series of tomograms shows the presence of subcortial ischemic focus in the right parietotemporal section with the sizes 2.4 x 1.8 x 3.2 cm with expressed perifocal edema, compression of the right lateral ventricle with displacement of midline structures up to 4.0 cm.

Lipid profile on admission: total blood cholesterol - 8.2 mmol/L; HDL - 1.3 mmol/L; LDL - 4.9 mmol/L; triglycerides - 1.82 mmol/L; atherogenicity coefficient - 5.3.

On the background of performed standardized antiaggregant, neuroprotective, and antioxidant therapy, Cellex medication was prescribed to the patient since the end of the first day, subcutaneously, 1 ml - 0.1 mg once per day for 10 days and repeated course in 21 days.

Three days later, apparent regress of the focal neurologic deficit was noted, as well as the increase of the range of motions on the left hand up to 4.5 scores by the 10th day, speech restoration by the 14th day.

Lipid profile on discharge: total blood cholesterol - 6.2 mmol/L; HDL - 1.4 mmol/L; LDL - 3.7 mmol/L; triglycerides - 1.6 mmol/L; atherogenicity coefficient - 3.4.

The patient was discharged for neurologist's supervision according to the place of residence on the 25th day with the following recommendations: continuation of Cellex intake subcutaneously, intake of the antiaggregant Thrombo ASS 100 mg before night, Ednyt 5 mg x 2 times per day, Lipostabil - 2 drops x 3 times/day; giving up smoking, consulting a vascular surgeon, supervision of neurologist according to the place of residence, lipid profile control, MRI with vascular mode in 6 months.

After discharge, the patient continued the course of Cellex, upon completion of which he took the medication intranasally by 2 drops in each nasal duct in the morning for 14 days with repeated course in 3 weeks; Thrombo ASS and Ednyt were taken regularly in recommended doses. The patient returned to work within 3.5 weeks after discharge.

Consultation of the vascular surgeon: stenting of the right internal carotid artery was offered, the patient refused.

Control lipid profile after 6 months: total blood cholesterol - 4.8 mmol/L; HDL - 1.46 mmol/L; LDL - 3.2 mmol/L; triglycerides - 1.38 mmol/L; atherogenicity coefficient - 2.3.

MRI series shows the formation of a liquor cyst in the right parietotemporal section with the sizes 1.8 x 0.9 x 2.2 cm with perifocal edema, compression of the lateral ventricle with displacement of midline structures is not detected; in the vascular mode, thickening of the vessel wall of the right internal carotid artery is registered with stenosis reduction down to 52%.

The patient is socially adapted, 6 months after discharge from the hospital he reduced the dose of cigarettes to 0.5 pack per day. He continued the prescribed therapy with the permanent intake of Thrombo ASS and Ednyt in the recommended doses and the course of treatment by Cellex once per 4 months subcutaneously, 1 ampoule (which contains 0.1 mg/ml of medication in 1 ml of the carrier (diluent, for example, buffer solution) for 10 days, and two 14-day courses intranasally - 2 drops in each nasal duct in the morning, with 2-2.5-week intervals between the courses of treatment.

Control of lipid profile after 12 months after discharge: total blood cholesterol - 4.2 mmol/L; HDL - 1.46 mmol/L; LDL - 2.7 mmol/L; triglycerides - 1.42 mmol/L; atherogenicity coefficient - 1.9.

MRI series shows a persisting liquor cyst in the right parietotemporal section without size change and compression on the adjacent structures; in the vascular mode, thickening of the vessel wall of the right internal carotid artery is registered with stenosis reduction down to 44%.

Therefore, regular Cellex intake by course doses, combining injection and intranasal methods of drug administration, showed an apparent antiatherogenic action of the drug with a reduction of stenosis intensity, thickening of the atherosclerotic plaque by means of activation of the lipid metabolism directly in it and in the artery's subendothelial layer.

### Example 2. Drug influence on the development of aortic wall explants of rats.

Experiments were carried out on the fragments of the vascular aortic wall of Wistar rats weighing 200-250 g. Medium for explants culturing consisted of 35% Eagle solution, 25% veal fetal deactivated serum, 35% Hanks' solution, 5% chicken embryonic extract, then glucose (0.6%), insulin (0.5 units/ml), penicillin (100 units/ml), glutamine (2 mM) were added to the medium. A section of vascular aortic wall was fragmented by eye scissors into explants sized 1-1.5 x 0.5-1.0 x 0.5-1.5 cm, and 10-15 pieces of them were placed into culture medium on the collagen-coated surface in Petri dishes in a thermostat at 36.7°C for 2 days. PPC and PPPC were added into experimental medium in the concentrations of 10, 50, 100, 200 and 400 mg/ml. The criterion of biological activity was the area index (AI) - the ratio of total explant area including the growth zone to the initial area of the fragment of the vascular wall. AI values were expressed in percents, control AI value was taken for 100%.

Study results: within 1 day of culturing, the explants spread on the collagen-coated surface, and ejection of proliferating and migrating cells along the explant's periphery started. On the 3rd day, a significant dose-dependent increase of the explants' AI by 20%, 30%, 65%, 45%, 20% was noted in contrast to the control values, with PPC concentrations equal to 10, 50, 100, 200 and 400 mg/ml respectively, and by 25%, 45%, 90%, 70%, 40% in contrast to the control values, with PPPC concentrations equal to 10, 50, 100, 200 and 400 mg/ml respectively. Upon examination of the growth of the vascular aortic wall explants within the culturing periods up to 7 days, the stimulating actions of PPC and PPPC in the same concentrations had similar ratios in contrast to the control.

Therefore, the drugs produced a tissue-specific effect in relation to the tissue of the vascular aortic wall, which was demonstrated by the growth stimulation of the explants.

### Example 3. Effectiveness of the drug among patients having cerebral atherosclerosis.

A study of the clinical effectiveness of Cellex was carried out with the participation of 30 patients aged 62-78, suffering from cerebral atherosclerosis. The patients had different clinical implications of cerebrovascular disorders corresponding to the nosology of residual encelopathy of vascular genesis of degree II-III, manifested in the form of memory and attention concentration impairments, affective lability, and mental deficiency. Patients from this group (additionally to the generally adopted treatment) received Cellex medication in the dose of 0.1 mg subcutaneously once per day for 14 days. The control group consisted of the patients with similar diagnosis, who received traditional standardized therapy (nootropics, antioxidants, antiaggregants). The control group included 12 patients receiving generally accepted treatment only.

During follow-up, patients' complaints were assessed, common and biochemical blood and urine assays were performed, and tests according to the international clinical scales were carried out (common functional disorders scale, Mattis dementia rating scale, CDR, mini-mental state examination (MMSE)). The effectiveness of the drug's influence on the brain's integrative functions was studied with the use of psychophysiological tests characterizing peculiarities of memory and thinking of the patients with cerebrovascular disorders: "Short-term visual memory for numbers", "Long-term memory for numbers" and "Determination of regularities".

It was found that the use of Cellex among patients having cerebral atherosclerosis contributed to the improvement of the general state, reduction of emotional lability, stabilization of mood, and mitigation of anxiety. The patients noted an improvement of their memory and attention concentration.

The most informative laboratory information was represented by the data of biochemical blood assay - lipid profile (see the Tables).

The use of the drug promoted a significant reduction in the blood's total cholesterol level as compared with this indicator prior to the treatment and after treatment with generally accepted agents.

There was also a distinct tendency towards a reduction of the content of very low-density lipoproteins, which are the most atherogenic lipoproteins. This indirectly proves a normalizing effect of the drug on the metabolism in the cells of the vessel wall.

With regard to patients from the control group, who received the generally accepted therapy, neither significant changes of psychophysiological test values nor significant changes of the total score according to the used clinical scales were detected.

With regard to patients from the basic group, who received treatment with the use of Cellex, significant changes of test values and clinical scales were detected in contrast to the values prior to treatment and values of the control group (see the Tables). This was demonstrated by positive dynamics of the change of cognitive functions characterizing volume and accuracy of visual information ("Short-term visual memory for numbers"), accuracy of memorizing, storage and reproduction of information (operative memory, OM), and logical thinking (ability to make judgments).

Moreover, patients from the basic group showed a significant increase in stability and concentration of attention, speed of information processing, normalization of the main cortical processes, improvement of short-term memory, and even a tendency towards improvement of the long-term memory, verifiable improvement of values characterizing logical thinking, and reduction of sensomotoric reaction time. Changes of psychophysiological values characterize an intensification of the excitative process in the cortex of patients having cerebrovascular disorders, which proves improvement of blood supply to cerebral sections exposed to ischemia and a favorable effect of the drug on blood vessels' functions. During the examination of clinical effectiveness of Cellex use, no adverse effects or complications were detected.

Therefore, results of the conducted clinical study give evidence of the medical effectiveness of the drug used in a therapeutically effective dose equal to 0.1 mg subcutaneously, once per day for 14 days, and of reasonability of its use in complex treatment of arterial atherosclerosis.

**Table 3.**

| Parameter | Prior to treatment | Control group after treatment | Group after treatment with Cellex |
|---|---|---|---|
| Total cholesterol, mmol/L | 8.4 ± 0.6 | 6.8 ± 0.4 * | 6.0 ± 0.2 * # |
| Cholesterol of very low density lipoproteins, mmol/L | 1.30 ± 0.07 | 1.14 ± 0.06 | 0.86 ± 0.08 * |
| Triglycerides, mmol/L | 4.7 ± 0.6 | 4.4 ± 0.6 | 4.3 ± 0.5 |
| Glucose, mmol/L | 5.3 ± 0.4 | 5.6 ± 0.5 | 5.4 ± 0.3 |

| | | | |
|---|---|---|---|
| * - P < 0.05 in contrast to the value prior to treatment; # - P < 0.05 in contrast to the value after treatment with the use of generally accepted agents. | | | |

**Table 4.**

| Parameter | Prior to treatment | After treatment with generally accepted agents | After treatment with the drug |
|---|---|---|---|
| Volume of short-term visual memory for numbers, | 6.4 ± 0.27 | 6.8 ± 0.32 | 8.7 ± 0.43 * |
| Operative memory | 23.4 ± 0.7 | 25.0 ± 0.36 | 31.3 ± 0.28 * # |
| Volume and resistance of operative memory | | | |
| Long-term memory for numbers, volume and resistance | 5.6 ± 0.16 | 5.9 ± 0.25 | 6.1 ± 0.18 |
| Determination of regularities, consistence of judgments (number of correct answers) | 34.5 ± 0.41 | 37.1 ± 0.29 | 44.2 ± 0.31 * |
| Total clinical score according to the common functional disorders scale | | | |
| Total clinical scale according to Mattis dementia rating scale | | | |
| Total clinical scale according to dementia scale (CDR) | | | |
| Total clinical scale according to mini-mental state examination scale (MMSE) | | | |

| | | | |
|---|---|---|---|
| * - P < 0.05 in contrast to the value prior to treatment; # - P < 0.05 in contrast to the value after treatment with the use of generally accepted agents. | | | |

### Example 4. Effect of Cellex medication on the atherogenic properties of blood serum

The impact of Cellex on the cholesterol outflow identified in the model in vitro was confirmed in the model ex vivo.

Table 5 represents data received from ex vivo model [5].

Volunteers took PPC - Cellex at once in the form of an intranasal spray in complex with polynucleotides in the form of sodium salts of DNA - PPPC, 400 µg of a 0.1% solution (per total protein and DNA) - 2 injections of 200 µg in each nasal duct in the morning, the drug for comparison was Verapamil in the dose of 400 mg; the control group received intranasally 50 mM of glycine-phosphate buffer solution with the same pH (7.8) and with the same regimen. Blood was taken prior to drug or control intake (time "0") and 1, 2, 4, and 8 hours after the intake. Samples were prepared from the bloodserum, and serum samples were added to the primary culture of atherosclerotic cells. A significant reduction of intracellular cholesterol content was observed in case of serum addition to the culture, which was prepared from blood taken 1 hour after single intake and 2 hours after Verapamil intake (Table 3). The same effect was demonstrated by serums taken 4, 8 and 12 hours after intake of the drugs. Consequently, starting from the 1 st hour after single intake of the claimed drug and from the 2nd hour after Verapamil intake, blood serum acquires antiatherosclerotic properties manifested in the contribution to reverse cholesterol transport from atherosclerotic cells. Antiatherosclerotic properties of serum are preserved for at least 12 hours after Verapamil intake and up to 18 hours after intake of the claimed drug.

**Table 5. Comparative table of the antiatherogenic action of Cellex and Verapamil in the ex vivo model.**

| Time between drug application and blood sampling (hr) | Intracellular cholesterol content, % of control - 221 ± 13 µg of cholesterol per 1 mg of cellular protein | | |
|---|---|---|---|
| | 50 mM Gly-Ph-buffer | Verapamil | Cellex |
| 1 | 102 ± 12 | 97 ± 10 | 82 ± 6* |
| 2 | 98 ± 8 | 84 ± 6 * | 75 ± 8 * |
| 4 | 92 ± 10 | 77 ± 8 * | 64 ± 10 ** |
| 8 | 104 ± 12 | 85 ± 4 * | 72 ± 8 * |
| 12 | 101 ± 6 | 90 ± 6 * | 86 ± 6 * |
| 18 | 98 ± 8 | 104 ± 4 | 90 ± 4 * |

| | | | |
|---|---|---|---|
| Notes: Control cells extracted from atherosclerotic lesion contained 221 ± 13 µg of cholesterol per 1 mg of cellular protein; this level was accepted as 100%; * - significant reduction of intracellular cholesterol level, p < 0.05; ** - significant reduction of intracellular cholesterol level, p < 0.01. | | | |

Thus, modifiers of biological activity - protein-polypeptide fractions of embryonic brain tissue of the hoofed livestock alone and together with DNA polynucleotides from the milts of salmons contributed to cholesterol outflow from atherosclerotic cells in the model in vitro, thus inducing a significant reduction of the intracellular cholesterol content in the cultured cells of the atherosclerotic lesion of the human's aorta (Table 5) more intensively than studied calcium antagonists. Besides, Cellex and the Cellex variant with polynucleotides along with calcium antagonists suppressed the proliferative activity of atherosclerotic cells of the vessel wall.

Some more examples are provided below regarding the embodiment of the method for the prevention and treatment of atherosclerosis of arterialvessels with diseases caused by atherosclerosis of major and peripheral vessels of the brain, heart, lower extremities, and aorta, in patients.

These examples illustrate but do not limit the scope claimed in the method of the invention.

According to all provided data, the claimed method for the treatment and prevention ensures a high cholesterol outflow from atherosclerotic cells, i.e. possesses an increased effectiveness for the treatment and prevention of atherosclerosis and is deemed to be industrially applicable.

The method is implemented as follows.

### Example 6.

The patient with atherosclerosis was examined by the method of ultrasound scanning of major vessels, which allowed detecting the presence of an atherosclerotic process with stable progression. The following was prescribed:
During one procedure - subcutaneously or intramuscularly, 0.05 - 1.0 mg of PPC in 1.0-5.0 ml of pharmaceutically acceptable diluent is administered in the form of 8-20 injections, 1-2 times daily, wherein the duration of a course is 8-14 days.

Additionally, a drug from the group of the statins (Atorvastatin 10 mg daily or Simvastatin 20 mg daily) or other anti-sclerotic substances described above are prescribed, for example, substances selected from the group, comprising sequestrants and hypolipidemic drugs.

The treatment is deemed to be successful if the control study (for example, ultrasound scanning of major vessels) shows that the volume of atherosclerotic lesion was reduced by 30-50%. In case of an insufficient effect, it is reasonable to repeat the treatment course.

### Example 7.

Patient Ch., 54 years old. Diagnosis: hypertensive disease of stage II, 2nd degree of AP increase, risk 3. Atherosclerosis of both common carotid arteries and right femoral artery. N 0.

During the examination on 30.05.2003 by the method of ultrasound scanning of major vessels using Hewlett Packard Sonos 4500, 3 atherosclerotic plaques were detected: in the right carotid artery, bifurcation area (1.20 x 0.170 cm in longitudinal projection), left common carotid artery (1.48 x 0.161 cm in longitudinal projection) and right femoral artery in the bifurcation area (2.34 x 0.245 x 0.201 cm in longitudinal projection), the last has been detected since 2001, and its is a stable, electron-dense plaque.

Treatment followed, comprising: During one procedure, subcutaneously or intramuscularly, 0.05 - 1.0 mg of protein/peptide-polynucleotide complex (hereinafter - PPPC) in 1.0-5.0 ml of pharmaceutically acceptable diluent is administered in the form of 8-20 injections, 1-2 times daily, wherein duration of the course is 8-14 days, with the ratio between PPC and polynucleotides of fish milt equal to 0.1-2 : 0.5-2 respectively; significant reduction of atherosclerotic plaques of both carotid arteries was noted, where residual changes were also observed in the form of intima thickening without luminal occlusion: in the right carotid artery, in the bifurcation area (0.884 x 0.135 cm in longitudinal projection), in the left common carotid artery (0.648 x 0.132 cm in longitudinal projection)/ In the right femoral artery in the bifurcation area, no significant changes of plaque sized occurred (2.67 x 0.172 x 0.275 cm in longitudinal projection). The total treatment effectiveness according to the results of the ultrasound scanning of major vessels amounted to about 17%.

### Example 8.

Patient P., 57 years old. Diagnosis: hypertensive disease of stage II, 3rd degree of AP increase, risk 3. Obliterating atherosclerosis of both common carotid arteries with both vessels constricted up to 50%. N I.

During the examination on 22.05.2003 by the method of ultrasound scanning of major vessels using Hewlett Packard Sonos 4500, atherosclerotic lesions were detected with 44% (on the right) and 55% (on the left) stenosis of the internal carotid arteries: in the left internal carotid artery, bifurcation area (thickness - 0.193 cm), left common carotid artery (0.403 x 0.604 cm in transverse projection), in the right internal carotid artery, bifurcation area (0.369 x 0.875 cm in transverse projection), and right common carotid artery (1.23 x 0.201 cm in longitudinal projection).

A treatment course followed, comprising: During one procedure, intramuscularly by drops or slow bolus, 0.025 - 0.7 mg of PPC in 5.0-500.0 ml of pharmaceutically acceptable diluent is administered, 1-2 times daily, wherein course duration is 5-12 days.

Upon this course of treatment, the following was detected: 4 areas of atherosclerotic lesion of the carotid arteries with a maximum stenosis level up to 45% (on the left) and an apparent reduction of the volume of atherosclerotic lesion of the right common carotid artery with 30% stenosis, i.e. reduction of stenosis level amounted to 14% on the right and 10% on the right from the size of the vessel lumen; in the left internal carotid artery, bifurcation area (thickness - 0.207 cm - without dynamics), left common carotid artery (0.403 x 0.763 cm - without dynamics), in the right internal carotid artery, bifurcation area (0.193 x 0.745 cm - significantly (by 55%) smaller than the initial size, and right common carotid artery (1.21 x 0.191 cm in longitudinal projection).

The second treatment course was performed, comprising the following: during one procedure, intramuscularly by drops or slow bolus, 0.025 - 0.7 mg of PPPC in 5.0-500.0 ml of pharmaceutically acceptable diluent is administered, 1-2 times daily, wherein the course duration is 5-12 days, or during one procedure, intranasally, 0.001 - 0.1 mg of PPC medication in 0.05-1.2 ml of pharmaceutically acceptable diluent is administered in the form of 2-3 injections or 2-4 drops in each nasal duct, 1-2 times daily, wherein the course duration is 10-14 days.

### Industrial applicability

The therapeutic drug PPC or PPPC is administered together with other antiatherosclerotic substances selected from the group comprising bile acids sequestrants, inhibitors of cholesterol synthesis in the liver (statins), additives influencing the cholesterol absorption, catabolism and clearance, and hypolipidemic drugs, in therapeutically effective doses.

Results: In the left internal carotid artery, bifurcation area (thickness - 0.202 cm - without dynamics), left common carotid artery (0.159 x 0.717 cm - reduction by more than 50%), in the right internal carotid artery, bifurcation area (0.190 x 0.483 cm - significantly, almost by 72% smaller than the initial size), and right common carotid artery (0.833 x 0.176 cm in longitudinal projection - reduction by 45%).

In a similar way, positive dynamics were observed in the improvement of the patient's state, which demonstrated the effectiveness of the method for the treatment of atherosclerosis upon implementing specified different course treatment regimens with the use of the specified drug.

Bibliography:
1. American Heart Association. Heart disease and stroke statistics - 2013. Dallas, American Heart Association.
2. Marcovina S.M., Crea F., Davignon J. et al. 2007. Biochemical and bioimaging markers for risk assessment and diagnosis in major cardiovascular disease: a road to integration of complementary diagnostic tools. J. Int. Med. 26: 214-234.
3. Vikhert A.M., Zhmurkin V.P., Zhuravleva A.I., Ilyinskiy B.V., Myasnikov L.A., Rabkin I.H., Rakhalsky Y.E., Sternberg E.Y. Atherosclerosis. J. "Ordo Deus", 2010, http://www.ordodeus.ru/Qrdo Deus12Ateroskleroz.html
4. Andreeva E.R., Mikhaylova I.A., Pugach I.M., Orekhov A.N. 1999. Cellular composition of atherosclerotic lesions of human aorta. Angio. Vascul. Surg. 5: 6-26.
5. A.N. Orekhov, I.A. Sobenin, V.A. Orekhova, A.A. Melnichenko, V.A. Myasoyedova, N.M. Mukhamedova, D.D. Sviridov, V.P. Karagodin, 2011. Cellular models for search of substances contributing to reverse cholesterol transport. J. Physiotherapy. B. 12, 19.10.1 1.
6. Nikiforov N.G., Grachev A.N., Lebedeva A.O., Orrekhov A.N., Sobenin I.A. Test system for the study of pharmacological agents influence on monocytes activation in human blood. J. Modem world, nature and human. 2011 2(1): 108 1 10.
7. D.A. Kharkevich 2010. Pharmacology: textbook - 10th ed., amended, revised and enlarged - 452 p.
8. Material from the medical website Surgeryzone© 2011-2013. Medications reducing cholesterol level. http://suraeryzone.net/informaciya-po-kardiologii/lekarstvennye-preparaty-snizhayushhie-uroven-xolesterina
9. Lopukhin Y.M. et al. Cholesterolosis, - Moscow. Medicine, 1983, p. 279
10. Gainer J.L. et al. - Experientia, 1992, v. 31, No. 5, p. 548-549
11. RU 2095059, 10.11.1997;
12. RU 2445106, 20.03.1912;
13. RU 2485132, 20.06.2013;
14. Mukhamedova N.M., Sviridov D.D., Karagodin V.P., Melnichenko A.A., Myasoyedova V.A., Orekhova V.A., Sobenin I.A., Orekhov A.N. Study of the influence of proteins potentially involved into cholesterol outflow on cholesterol export from macrophages. J. Problems and prospects of modern science. 2011 3(1): 68-73.

## Claims

1. Use of a protein/peptide complex (hereinafter - PPC) produced from embryonic nervous tissue or quickly frozen embryonic brain of hoofed livestock, influencing the reverse cholesterol transport from the vessel wall and the activation profile of monocytes of patients with expressed atherosclerosis of major vessels or with a predisposition to cardiovascular diseases, for the treatment and prevention of atherosclerosis, comprising negatively charged, neutral proteins and polypeptides, with the molecular weights from 0.5 to 200 kDa, or in its combination with polynucleotides in the form of sodium salts of DNA extracted from the milts of sturgeon and salmon families, in the form of a protein/peptide-polynucleotide complex for the prevention and treatment of arterial vessels with atherosclerosis and diseases caused by atherosclerosis of major and peripheral vessels of the brain, heart, lower extremities, and aorta, in patients, administered parenterally or intranasally in therapeutically effective daily and course doses, and regulating the metabolism of cholesterol, cholesterol esters, triglycerides, and phospholipids in subendothelial connective tissue and smooth muscle cells of arterial vessels.

2. Method for prevention and treatment of atherosclerosis of arterial vessels and diseases caused by atherosclerosis of major and peripheral vessels of the brain, heart, lower extremities, and aorta, in patients, comprising the parenteral or intranasal administration to patients of a drug comprising a protein/peptide complex (hereinafter - PPC) in combination with the pharmaceutically acceptable carrier in therapeutically effective daily or course doses, as a drug regulating the metabolism of cholesterol, cholesterol esters, triglycerides, and phospholipids in subendothelial connective-tissue and smooth-muscle cells of arterial vessels; wherein, the protein/peptide complex (PPC) is used with a total protein content equal to 0.05-4.2 mg/ml, produced from embryonic nerve tissue of hoofed livestock, comprising tissue-specific negatively charged, slightly acidic, neutral proteins and polypeptides classified as growth and differentiation factors, signaling molecules determining its biological activity, with molecular weights ranging from 0.5 to 200 kDa, wherein at least 70% of the total protein amount has a molecular weight ranging from 20 to 160 kDa, having a specific set of bands at denaturing gel electrophoresis ("SDS-Page") in 5% polyacrylamide gel, an isoelectric point from 4.2 to 8.4, and an absorption peak at a wavelength (215±5 nm) in the ultraviolet spectrum recorded at the wavelengths ranging from 200 to 500 nm.

3. Method for prevention and treatment of atherosclerosis of arterial vessels and diseases caused by atherosclerosis of major and peripheral vessels of the brain, heart, lower extremities, and aorta, in patients, comprising the parenteral or intranasal administration to patients of a drug containing a protein/peptide complex (hereinafter - PPC) in combination with the pharmaceutically acceptable carrier in therapeutically effective daily or course doses, as a drug regulating the metabolism of cholesterol, cholesterol esters, triglycerides, and phospholipids in subendothelial connective-tissue and smooth-muscle cells of arterial vessels; wherein, the protein/peptide complex used, is produced from homogenate of quickly-frozen embryonic brain tissues of hoofed livestock, and comprises negatively charged and neutral proteins and polypeptides with a molecular weights from 5 to 200 kDa, and a fraction content of at least 80% with an average molecular weight from 10 to 120 kDa, a total protein content of 0.05 - 4.2 mg/ml, with the maximum absorption peak of a UV spectrum of the solution at a wavelength of 280±5 nm, with a peak present at the wavelength 274-284 nm in the UV-visible spectrum, and the presence of specific bands within the pl range from 4.2 to 8.4 at isoelectric focusing in 5% polyacrylamide gel.

4. Method for prevention and treatment of atherosclerosis of arterial vessels and diseases caused by atherosclerosis of major and peripheral vessels of the brain, heart, lower extremities, and aorta, in patients, comprising the administration of a therapeutic drug containing a protein/peptide complex according to claim 2 or 3, differing in the fact that the therapeutic drug additionally comprises polynucleotides (as an active immune modeling and stabilizing component) in the form of sodium salts of DNA produced from the milts of sturgeon and salmon families, **characterized by** the molecular weight from 12 to 660 kDA (18-1000 base pairs) in equal or necessary therapeutically sufficient daily and course dose, with their total concentration in the therapeutic drug of protein/peptide-polynucleotide complex (PPPC) equal to approximately 0.05-3.0 mg/ml.

5. Method for prevention and treatment of atherosclerosis of arterial vessels and diseases caused by atherosclerosis of major and peripheral vessels of the brain, heart, lower extremities, and aorta, in patients, according to claim 2 or 3, differing in the fact that the drug additionally comprises the immune modeling and stabilizing component in equal or necessary therapeutically sufficient daily and course dose, represented as hydrolyzed polynucleotides in the form of sodium salts of DNA produced from the milts of sturgeon and salmon families, with the chain length of the polynucleotides ranging from approximately 1000 to 7000 nucleotides, and their concentration in the drug being approximately 0.05-3.0 mg/ml (milligram per milliliter).

6. Method for prevention and treatment of atherosclerosis of arterial vessels and diseases caused by atherosclerosis of major and peripheral vessels of the brain, heart, lower extremities, and aorta, in patients, according to the claim 2 or 3, differing in the fact that the drug contains a pharmaceutically acceptable diluent, comprising saline or buffer-saline solutions as a pharmaceutically acceptable carrier, and, possibly, the excipients.

7. Method according to claim 2 or 3, differing in that during one procedure, subcutaneously or intramuscularly, from 0.05 to 1.0 mg of PPC in 1.0-5.0 ml of pharmaceutically acceptable diluent is administered in the form of 10-14 injections with the course being repeated after 7-10 days.

8. Method according to one of claims 4 or 5, differing in that during one procedure from 0.05 to 1.0 mg of PPPC in 1.0 - 5.0 ml of pharmaceutically acceptable diluent is administered subcutaneously or intramascularly in the form of 8-20 injections, 1-2 times daily, over a course of 8-14 days, where the ratio between PPC and polynucleotides from the fish milt can be 0.1-2 : 0.5-2 respectively;

9. Method according to claim 2 or 3, differing in that during one procedure from 0.025 to 0.7 mg of PPC in 5.0 - 500.0 ml of pharmaceutically acceptable diluent is administered intravenously by drops or slow bolus, 1-2 times daily, wherein the course is 5-12 days.

10. Method according to one of claims 4 or 5, differing in that during one procedure from 0.025 to 0.7 mg of PPPC in 5.0 - 500.0 ml of pharmaceutically acceptable diluent is administered intravenously by drops or slow bolus, 1-2 times daily, wherein the course is 5-12 days.

11. Method according to claim 2, differing in that during one procedure from 0.001 to 0.1 mg of PPC in 0.05 - 1.2 of pharmaceutically acceptable carrier-diluent is administered in the form of 2-3 injections or 2-4 drops in each nasal duct, 1-2 times daily, wherein the course is 10-14 days.

12. Method according to claim 5, differing in that during one procedure from 0.001 to 0.1 mg of PPPC in 0.05 - 1.2 ml of pharmaceutically acceptable diluent is administered in the form of 2-3 injections or 2-4 drops in each nasal duct, 1-2 times daily, the course being 10-14 days.

13. Method according to either claim 2 or 3, differing in that the course of the treatment is performed by subcutaneous administration of PPC once per 4 months in the dose of 0.05-0.1 mg in 1.0-5.0 ml of carrier (diluent), one corresponding injection for 10 days, and two 14-day courses are performed by intranasal administration of 2 drops in each nasal duct in the morning with an interval of 2.0-2.5 weeks between the courses.

14. Method according to claim 4 or 5, differing in that a PPC or PPPC drug is administered together with other antiatherosclerotic drugs selected from the group comprising bile acids sequestrants, inhibitors of cholesterol synthesis in the liver (statins), additives influencing the cholesterol absorption, catabolism, and clearance, and hypolipidemic drugs in therapeutically effective doses.

15. Method according to claim 4 or 5, differing in that systematic course administration of a PPC or PPPC drug is performed in 4-12 courses per year, with an interval of 10-56 days between the courses, depending on the intensity of the atherosclerotic process, its localization, the existence of any complications, and their consequences.

16. Method according to claim 4 or 5, differing in that upon preventive treatment of atherosclerosis, a systematic course administration of a PPC or PPPC drug is performed in the form of 2-6 courses per year, with intervals of 2-6 months between the courses, depending on the aggregation risk factors and genetic predisposition of the patient.

17. Method according to claim 2 and 3, differing in that upon the preventive treatment of vessels with atherosclerosis, a systematic course administration of a PPC or PPPC drug is performed in the form of 2-6 courses per year, with intervals of 2-6 months between the courses, depending on the aggregation risk factors and genetic predisposition of the patient, together with other antiatherosclerotic drugs selected from the group comprising bile acids sequestrants, inhibitors of cholesterol synthesis in the liver (statins), additives influencing the cholesterol absorption, catabolism, and clearance, and hypolipidemic drugs in therapeutically effective doses.
